Europäisches Patentamt

European Patent Office

Office européen des brevets

(19)

(11) Veröffentlichungsnummer: **0 022 144**
**B1**

(12) **EUROPÄISCHE PATENTSCHRIFT**

(45) Veröffentlichungstag der Patentschrift: **28.12.83**

(21) Anmeldenummer: **80100624.8**

(22) Anmeldetag: **07.02.80**

(51) Int. Cl.³: **A 61 M 16/00**

(54) Beatmungsanlage mit von Patientenwerten gesteuertem Beatmungsgerät.

(30) Priorität: **03.07.79 DE 2926747**

(43) Veröffentlichungstag der Anmeldung:
**14.01.81 Patentblatt 81/2**

(45) Bekanntmachung des Hinweises auf die Patenterteilung:
**28.12.83 Patentblatt 83/52**

(84) Benannte Vertragsstaaten:
**BE CH FR GB NL SE**

(56) Entgegenhaltungen:
**WO - A - 80/00917**
**DE - A - 2 728 779**
**DE - A - 2 744 327**
**DE - B - 2 446 281**
**DE - B - 2 505 670**
**DE - B - 2 507 981**

**MEDICAL & BIOLOGICAL ENGINEERING &
COMPUTING, Band 17, Nr. 4, Juli 1979, London
P. COLLINS et al. "Apparatus for the
Servocontrol of Arterial Oxygen Tension in
Preterm Infants"**

(73) Patentinhaber: **Drägerwerk Aktiengesellschaft
Moislinger Allee 53-55
D-2400 Lübeck 1 (DE)**

(72) Erfinder: **Schulz, Volker, Prof. Dr. med.
Hans-Böckler-Strasse 86
D-6500 Mainz 1 (DE)**
Erfinder: **Kunke, Stefan, Dr. med.
Adelheidstrasse 51
D-6200 Wiesbaden (DE)**
Erfinder: **Heim, Ulrich, Dr.
Klosterstrasse 19
D-2067 Reinfeld (DE)**

(56) Entgegenhaltungen:
**IEEE TRANSACTIONS ON BIOMEDICAL
ENGINEERING, Band BME-24, Nr. 4, Juli 1977
New York D.R. WESTENSKOW et al.
"Instrumentation for Measuring Continuous
Oxygen Consumption of Surgical Patients"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Beatmungsanlage mit von Patientenwerten gesteuertem Beatmungsgerät

Die Erfindung betrifft eine Beatmungsanlage mit von Patientenwerten gesteuertem Beatmungsgerät entsprechend dem Gattungsbegriff des Anspruches 1.

Die Beatmung mit Beatmungsgeräten soll eine genügende Sauerstoffversorgung des Körpergewebes sichern. Angestrebt wird eine optimale Sättigung des Blutes. Erfolgt die Sauerstoffzufuhr jedoch unter einer zu hohen Inspirationskonzentration $O_2(FiO_2)$ im zugeführten Atemgas, so kann es, insbesondere bei Langzeitbeatmung, zu Veränderungen des Lungengewebes, des Blutkreislaufes sowie toxischen Wirkungen kommen. Auch werden sich anbahnende Insuffizienzen durch das reichliche Angebot überdeckt und einer frühzeitigen Diagnose entzogen. Deshalb sollte $FiO_2$ so klein wie möglich gehalten werden.

Eine bekannte Beatmungsanlage besitzt eine Einrichtung zum Steuern und Regeln der Ausgangsgrößen eines Beatmungsgerätes. Das Beatmungsgerät wird in Abhängigkeit von physiologischen Parametern eines Patienten sowie der Analysenwerte der gasförmigen Komponenten des Blutes gesteuert.

Hierzu gelangen die am Patienten in einem Lungefunktions-Meßgerät und Analysator gewonnenen Meßwerte sowohl an einen Rechner als auch an eine Hauptsteuereinheit. Die Hauptsteuereinheit beeinflußt über den zum Beatmungsgerät gehörenden Gasmischer die Zusammensetzung des Gasgemisches und über eine Steuer- und Regeleinrichtung die anderen Ausgangsgrößen des Beatmungsgerätes. Ein Steuerteil löst über die Hauptsteuereinheit in bestimmten Zeitintervallen für eine kurze Zeitdauer eine Änderung der Ausgangsgrößen des Beatmungsgerätes aus. Aus den Signalen, die im Verlauf der Änderung vom Patienten erhalten werden, werden von dem Rechner Steuersignale für die Hauptsteuereinheit gebildet. Die Hauptsteuereinheit steuert dann die Ausgangsgrößen des Beatmungsgerätes. Möglichkeiten zur Optimierung bestimmter Werte wie z.B. $PaO_2$ oder $FiO_2$ ergeben sich aus dieser bekannten Beatmungsanlage nicht. Sie stellt lediglich Faktoren fest, die für die Durchführung eines noch anzugebenden Programmes von Nutzen sind. (DE—OS 27 44 327).

Ein bekanntes Verfahren und Vorrichtung zur Regelung der künstlichen Beatmung umfaßt Meßgeräte für die arteriellen Teildrücke von Sauerstoff ($PaO_2$) und Kohlensäure ($PaCO_2$) des Patienten, ein Beatmungsgerät mit Einstellmöglichkeit der Beatmungsparameter wie Sauerstoffkonzentration $FiO_2$, Atemfrequenz, Beatmungsvolumen, Kohlensäurekonzentration $FiCO_2$ und einen Rechner, der aufgrund der Signale der Meßgeräte und des Beatmungsgerätes eine Steuerung des Beatmungsgerätes zur automatischen Regelung und Optimierung der Beatmung vornimmt. Hierzu wird unter kontinuierlicher oder periodisch in Intervallen von wenigen Minuten durchgeführter Messung von $PaO_2$ und $PaCO_2$ in einem 1. Schritt als erster Einstellparameter die $FiO_2$ in vorgegebenen Grenzen variiert, bis eine empirische Beziehung E zwischen $PaO_2$, $PaCO_2$ und $FiO_2$ einen Minimalwert annimmt. In einem 2. Schritt werden nacheinander weitere Einstellparameter wie Beatmungsvolumen, Atemfrequenz, $FiCO_2$ variiert, derart, daß eine weitere Beziehung F zwischen $PaO_2$ und $PaCO_2$ einem Minimalwert annimmt.

Das Verfahren kann aber auch in einer in Abhängigkeit von dem am Ende des 1. Schrittes erreichten Wert $PaO_2$ differenzierten Weise durchgeführt werden. Wird beim Erreichen des minimalen Wertes $E_1$ der $PaO_2 \leq 110$ mm Hg (146 mbar), so wird die Verstellung von $FiO_2$ beendet und der erreichte Wert $FiO_2$ beibehalten. Dann schließt sich der beschriebene 2. Shritt zur Minimierung des Wertes F an.

Wird aber beim Erreichen des minimalen Wertes $E_1$ der $PaO_2 > 110$ mm Hg (146 mbar), wird die $FiO_2$ weiter verringert, bis $PaO_2 = 90...100$ mm Hg (120...133 mbar) erreicht. Ist der jetzige Wert $E_2$ kleiner als der zunächst erreichte Minimalwert $E_1$, so wird a) bei $PaO_2 \geq 90$ mm Hg (120 mbar) die $FiO_2$ um 5% verringert oder b) bei $PaO_2 < 90$ mm Hg (120 mbar) die $FiO_2$ um 5% erhöht, ehe der 2. Schritt zur Minimierung des Wertes F erfolgt. Ist dagegen $E_2$ größer als der zunächst erreichte Minimalwert $E_1$, so werden c) die bei Erreichen von $E_1$ herrschenden Einstellungen wiederhergestellt ehe der 2. Schritt zur Minimierung des Wertes F erfolgt.

Nach Durchführung der Minimierung des Wertes F unter Veränderung weiterer Einstellparameter wird der dann erreichte Wert $E_3$ errechnet. Ist $E_3$ kleiner als $E_2$, wird erneut entsprechend a) oder b) verfahren. Ist dagegen $E_3$ wiederum größer als $E_2$, so hat sich der Zustand des Patienten geändert, und das Verfahren muß von Anbeginn wiederholt werden.

Nachteilig ist, daß der Wert für $PaO_2$, der einen Fundamentalwert darstellt, dabei nicht festgelegt ist und sich während der Veränderung der Einstellparameter von seinem Optimalwert entfernen kann, ehe eine Korrektur erfolgt. Dies kann für den Patienten ungünstig sein. Da die empirischen Beziehungen jeweils mehrere Variable umfassen, ist mit der Minimierung des Gesamtwertes der Beziehung nicht gesichert, daß beim Patienten stets die optimale $O_2$-Sättigung des Blutes unter Anwendung der dafür geringstmöglichen $FiO_2$ eingehalten wird (DE—OS 27 28 779).

Aufgabe der Erfindung ist eine Beatmungsanlage, die eine größtmögliche Schonung des Patienten durch Aufrechterhaltung seines günstigsten arteriellen Partialdruckes $O_2$ ($PaO_2$)

durch Zufuhr der dafür geringstmöglichen Inspirationskonzentration $O_2$ (Fi$O_2$) erreicht.

Die Lösung der Aufgabe erfolgt gemäß dem Kennzeichen des Anspruches 1.

Durch die Minimierungseinrichtung wird die Fi$O_2$ unter Verstellung von Beatmungsparametern zu einem Minimum geführt, wobei der Pa$O_2$ durch den Regler ständig auf dem gewünschten Wert gehalten wird. Dies war bisher nicht möglich und ist vorteilhaft für den Patienten. Der selbsttätige Ablauf macht die Minimierung unabhängig von ständiger menschlicher Lenkung und damit überhaupt erst anwendbar.

Vorteilhafte Weiterbildungen ergeben sich aus den Ansprüchen 2—5. Die Heranziehung weiterer Patientendaten als zusätzliches Kriterium für die Beibehaltung von Parameteränderungen führt zu besonderer Schonung des Patienten, weil damit gesichert ist, daß im Verlauf der Minimierung der Fi$O_2$ diese weiteren Patientendaten bestimmte günstige Bereiche nicht verlassen. Auch die Ausstattung mit Warngeräten und dem Alarmgerät dient der Schonung und Sicherheit des Patienten sowie der Erleichterung für das Bedienungspersonal, indem das Auftreten und die Art von Abweichungen sowie eine nicht durch die Beatmungsanlage zu bewältigende Änderung des Patientenzustandes sofort erkennbar werden. Korrekturen können dann unverzüglich vorgenommen werden.

Ausführungsbeispiele der Erfindung sind in der Zeichnung in schematischer Darstellung gezeigt und im folgenden beschrieben. Es zeigen

Fig. 1 eine Grundausführung der Beatmungsanlage

Fig. 2 eine erweiterte Ausführung der Beatmungsanlage.

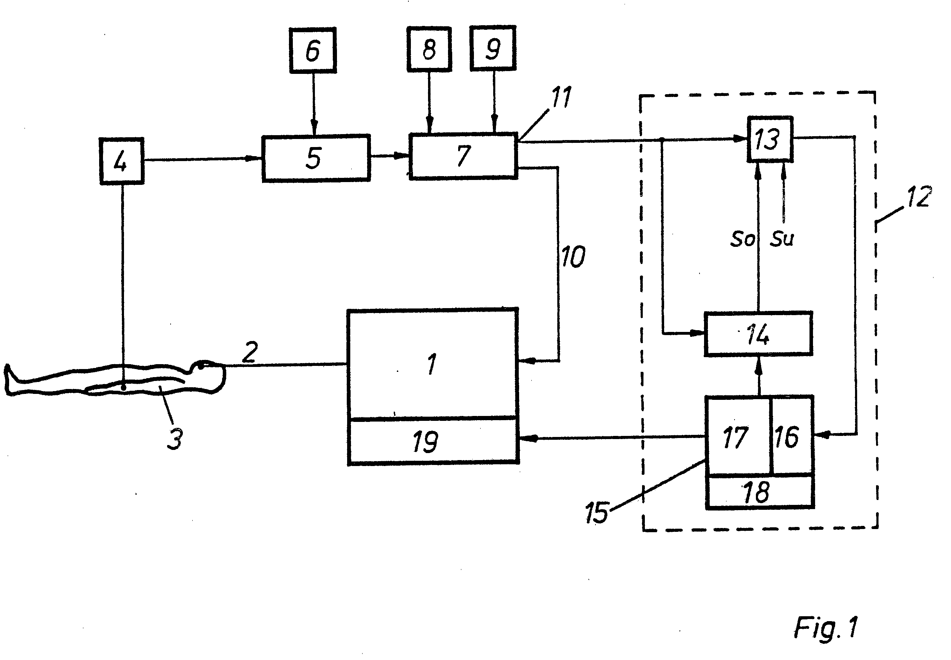

In Fig. 1 ist das Beatmungsgerät 1 über die Atemgasleitung 2 mit den Atemorganen des Patienten 3 verbunden. Der Pa$O_2$-Fühler 4 liegt dem Patienten 3 an und mißt dessen Pa$O_2$. Sein Signal gibt er an den Ist-Eingang des Reglers 5 weiter. Dessen Soll-Eingang erhält durch den Sollwertgeber 6 ein Sollsignal entsprechend einem gewünschten Pa$O_2$. Das Ausgangssignal steuert den Mischer 7. Dieser wird aus der Druckluftquelle 8 und der Sauerstoffquelle 9 vorsorgt. Das Atemgas gelangt über die Verbindungsleitung 10 zum Beatmungsgerät 1 und dann über die Atemgasleitung 2 zum Patienten 3. Der Regler 5 sorgt über die von ihm bestimmte Fi$O_2$ des Atemgases für die ständige Einhaltung des gewünschten Pa$O_2$ des Patienten 3. Der Mischer 7 gibt an dem Signalausgang 11 ein der augenblicklichen Fi$O_2$ entsprechendes Signal gleichzeitig an den Minimierungskomparator 13 und den Speicher 14 der Minimierungseinrichtung 12. Der Speicher 14 ist als Schwellwertgeber für den oberen Schwellwert So mit dem Minimierungskomparator 13 verbunden. Der untere Schwellwert Su des Minimierungskomparators 13 ist an einem nicht dargestellten Schwellwertgeber eingestellt. Der Ausgang des Minimierungskomparators 13 ist mit der Steuereinrichtung 15 und diese mit dem Speicher 14 und der Stellanordnung 18 verbunden.

Die Steuereinrichtung 15 umfaßt einen Flip-Flop 16, einen Mikroprozessor 17 für die Steuerung der Abläufe und einen Zeitgeber 18. Sie veranlaßt an der Stellanordnung 19 in vorgegebener Folge die Verstellung je eines Beatmungsparameters des Beatmungsgerätes 1, wie Tidalvolumen, Atemfrequenz, Minutenvolumen, zeitliches Strömungsmuster, endinspiratorische Pause, Amplitude der Atemgasströmung, endinspiratorischer Plateau-Druck, Spitzendruck, PEEP-Druck, Geräte-Totraum, Geräte-Compliance oder inspiratorische $CO_2$-Konzentration. Der jeweilige Beatmungsparameter wird dabei um einen Bruchteil, z.B. jeweils um 1/10, seines Verstellbereiches verstellt. Je nach Art des betreffenden Beatmungsparameters wird der Zeitgeber 18 durch den Mikroprozessor 17 auf eine bestimmte Taktzeit eingestellt.

Dabei ergibt sich der folgende Ablauf: Auf Taktung durch ihren Zeitgeber 18 setzt die Steuereinrichtung 15 ihren Flip-Flop 16 auf Ausgangssignal O. Gleichzeitig veranlaßt sie den Speicher 14, die augenblickliche Fi$O_2$ des Mischers 7 zu speichern und als So auf den Minimierungskomparator 13 zu geben. Ferner löst sie an der Stellanordnung 19 die Verstellung eines 1. Beatmungsparameters aus. Als Folge tritt eine Änderung der Fi$O_2$ ein, um den gewünschten Pa$O_2$ festzuhalten. Wenn während der durch den Zeitgeber 18 bestimmten Taktzeit das Signal des Mischers 7 im Bereich zwischen den Schwellwerten Su und So des Minimierungskomparators 13 liegt, gibt der Minimierungskomparator 13 ein O-Signal ab und der Flip-Flop 16 bleibt auf Ausgangssignal O. Nach Ablauf der Taktzeit wird die Richtung der erfolgreichen Verstellung des 1. Beatmungsparameters in dem Mikroprozessor 17 gespeichert. Dann wiederholt sich der beschriebene Ablauf unter Verstellung eines 2. Beatmungsparameters, wobei die gerade erreichte Fi$O_2$ als neuer Schwellwert So für den Minimierungskomparator 13 dient.

Führt eine Verstellung sofort oder innerhalb der Taktzeit zu der Überschreitung des So bzw. Unterschreitung des Su, so gibt der Minimierungskomparator 13 ein L-Signal ab und schaltet den Flip-Flop 16 auf Ausgangssignal L. Dies erfolgt auch bei einer nur vorübergehenden Über- bzw. Unterschreitung. Mit dem Flip-Flop 16 wird so ein stabiles Verhalten der Beatmungsanlage gesichert. Durch das Ausgangssignal L des Flip-Flop 16 wird die Steuereinrichtung 15 veranlaßt, die Verstellung an der Stellanordnung 19 wieder rückgängig zu machen, während die ursprüngliche Fi$O_2$ im Speicher 14 erhalten bleibt. Ergibt sich nach Ablauf einer weiteren Taktzeit keine Überschreitung des So bzw. Unterschreitung des Su,

well der Patient 3 seinen Ausgangszustand wieder erreicht hat, setzt die Steuereinrichtung 15 den Flip-Flop 16 wieder auf Ausgangssignal O. Dann verstellt die Steuereinrichtung 15 den Beatmungsparameter, dessen Verstellung hatte rückgängig gemacht werden müssen, in der der ursprünglichen Verstellung entgegengesetzten Richtung. Der Ablauf vollzieht sich erneut.

Denach geht die Steuereinrichtung 15 zum nächsten Beatmungsparameter über, bis deren Reihe durchlaufen ist. Dann beginnt ein neuer Zyklus, wobei jeder Beatmungsparameter in der Richtung verstellt wird, in der er beim vorhergehenden Zyklus zu einer Verringerung der $FiO_2$ betragen konnte.

In dieser Weise werden Parameteränderungen mit einer $FiO_2$ größer als vorher automatisch rückgängig gemacht. Lediglich Parameteränderungen mit einer $FiO_2$ kleiner als vorher bleiben erhalten und bilden mit ihrer $FiO_2$ den neuen oberen Schwellwert. Damit wird die $FiO_2$ bei konstantem $PaO_2$ zu einem Minimum geführt.

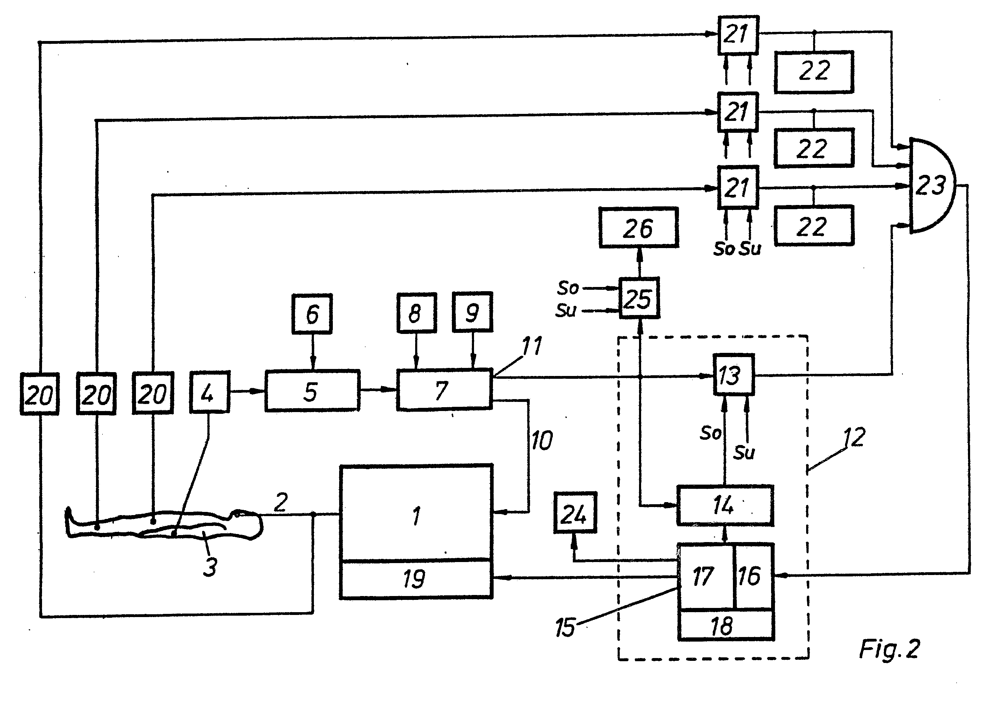

In der Ausführung nach Fig. 2 sind Fühler 20 zur Messung von weiteren Patientendaten an die Minimierungseinrichtung 12 angeschlossen. Der Fühler 20 für das Herzzeitminutenvolumen (HZV) und der weitere Fühler 20 für den arteriellen Partialdruck $CO_2$ ($PaCO_2$) liegen dem Patienten 3 an. Der weitere Fühler 20 für den Atemwegsdruck ($P_{air}$) ist in die Atemgasleitung 2 eingefügt. Die Signale der Fühler 20 gehen jeweils an die Fensterkomparatoren 21, an denen obere und untere Schwellwerte über nicht dargestellte Schwellwertgeber vorgegeben sind. Liegt das Signal des jeweiligen Fühlers 20 zwischen diesen Schwellwerten, dann gibt der zugehörige Fensterkomparator 21 ein O-Signal ab. Verläßt das Signal des Fühlers den Bereich zwischen den Schwellwerten, gibt der Fensterkomparator 21 ein L-Signal ab und löst an dem jeweils angeschlossenen Warngerät 22 eine Warnung aus. Die Signale der Fensterkomparatoren 21 und des Minimierungskomparators 13 sind parallel an das ODER-Gatter 23 geführt. Dieses gibt das Ausgangssignal O, wenn alle Eingänge Signal O führen; andernfalls ist das Ausgangssignal L. Das ODER-Gatter 23 ist mit dem Flip-Flop 16 der Steuereinrichtung 15 verbunden. Die Steuereinrichtung 15 ist mit der Stellanordnung 19, dem Speicher 14 und dem Alarmgerät 24 verbunden.

Zusätzlich ist mit dem Eingang des Minimierungskomparators 13 ein $FiO_2$-Fensterkomparator 25 mit angeschlossenem Warngerät 26 verbunden, dessen unterer (Su) und oberer (So) Schwellwert durch nicht dargestellte Schwellwertgeber dem Zustand des Patienten 3 entsprechend eingestellt sind. Verläßt das Signal des Mischers 7 den Bereich zwischen diesen Schwellwerten, so löst der $FiO_2$-Fensterkomparator 25 an dem Warngerät 26 eine Warnung aus.

Der Ablauf erfolgt sinngemäß wie bereits beschrieben: Auf Taktung durch ihren Zeitgeber 18 setzt die Steuereinrichtung 15 den Flip-Flop 16 auf Ausgangssignal O. Gleichzeitig veranlaßt sie den Speicher 14, die augenblickliche $FiO_2$ des Mischers 7 zu speichern und als So auf den Minimierungskomparator 13 zu geben. Ferner Löst sie an der Stellanordnung 19 die Verstellung eines 1. Beatmungsparameters aus. Wenn während der Taktzeit keine Überschreitung des So bzw. Unterschreitung des Su des Minimierungskomparators 13 und der Fensterkomparatoren 21 erfolgt und der Ausgang der ODER-Gatters 23 auf O-Signal bleibt, verbleibt auch der Flip-Flop 16 auf Ausgangssignal O. Nach Ablauf der Taktzeit wird die Richtung der erfolgreichen Verstellung des 1. Beatmungsparameters in der Steuereinrichtung 15 gespeichert. Dann wiederholt sich der beschrieben Ablauf unter Verstellung eines 2. Beatmungsparameters. wobei die gerade erreichte $FiO_2$ als neuer Schwellwert So für den Minimierungskomparator 13 dient.

Führt eine Verstellung sofort oder innerhalb der Taktzeit zu der Überschreitung des So bzw. Unterschreitung des Su des Minimierungskomparators 13 oder eines der Fensterkomparatoren 21, so entsteht am Ausgang des ODER-Gatters 23 ein L-Signal und schaltet den Flip-Flop 16 auf Ausgangssignal L. Dadurch wird die Steuereinrichtung 15 veranlaßt, die Verstellung wieder rückgängig zu machen, während die ursprüngliche $FiO_2$ im Speicher 14 erhalten bleibt. Ergibt sich nach Ablauf einer weiteren Taktzeit keine Überschreitung oberer bzw. Unterschreitung unterer Schwellwerte, weil der Patient 3 seinen Ausgangszustand wieder erreicht hat, setzt die Steuereinrichtung 15 den Flip-Flop 16 wieder auf Ausgangssignal O. Dann verstellt die Steuereinrichtung 15 den Beatmungsparameter, dessen Verstellung hatte rückgängig gemacht werden müssen, in der der ursprünglichen Verstellung entgegengesetzten Richtung. Der Ablauf vollzieht sich erneut.

Fall nach obiger Rückgängigmachung der erfolglosen Verstellung der Patient 3 seinen Ausgangszustand während der Taktzeit nicht wieder erreichen sollte, d.h. am Ausgang des ODER-Gatters 23 bleibt L-Signal stehen, muß angenommen, werden, daß den Maßnahmen des Gerätes ein davon unabhängiger Trend des Patienten zur Veränderung seiner Daten überlagert ist. In diesem Fall stellt die Steuereinrichtung 15 die gesamte Automatik ab und gibt über das Alarmgerät 24 optischen und akustischen Alarm.

**Patentansprüche**

1. Beatmungsanlage mit von Patientenwerten gesteuertem Beatmungsgerät, wobei dem Beatmungsgerät (1) aus einem Mischer (7), der seine Stellwerte aus einem über einen $PaO_2$-Fühler (4) am Patienten (3) und mit eingegebenem Sollwert gesteuerten Regler (5) erhält, das Atemgas mit darin bestimmter $FiO_2$ zugeführt

wird, dadurch gekennzeichnet, daß a) die $FiO_2$-Werte gleichzeitig einem Speicher (14) und einem Minimierungskomparator (13) einer Minimierungseinrichtung (12) anliegen, wobei der obere Schwellwert So des Minimierungskomparators (13) die vom Speicher (14) gehaltene bisherige $FiO_2$ ist, und b) eine Steuereinrichtung (15) der Minimierungseinrichtung (12), die einen Flip-Flop (16), einem Mikroprozessor (17) und einen Zeitgeber (18) enthält, an der Stellanordnung (19) des Beatmungsgerätes (1), um dort nacheinander Beatmungsparameter zu verstellen, angeschlossen ist.

2. Beatmungsanlage nach Anspruch 1, dadurch gekennzeichnet, daß an dem Flip-Flop (16) über ein ODER-Gatter (23) parallel zueinander Fühler (20) zur Messung von weiteren Patientendaten über Fensterkomparatoren (21) und der Minimierungskomparator (13) angeschlossen sind.

3. Beatmungsanlage nach Anspruch 1 und 2, dadurch gekennzeichnet, daß die Fensterkomparatoren (21) mit je einem Warngerät (22) verbunden sind.

4. Beatmungsanlage nach Anspruch 1 bis 3, dadurch gekennzeichnet, daß mit dem Eingang des Minimierungskomparators (13) ein $FiO_2$-Fensterkomparator (25) mit einem Warngerät (26) verbunden ist.

5. Beatmungsanlage nach Anspruch 1 bis 4, dadurch gekennzeichnet, daß an der Steuereinrichtung (15) ein Alarmgerät (24) angeschlossen ist.

## Revendications

1. Respirateur artificiel commandé par des valeurs mesurées sur le patient, dans lequel le gaz respiratoire, avec une concentration en oxygène $FiO_2$ déterminée, est amené au respirateur artificiel (1) par un mélangeur (7), qui reçoit ses valeurs de réglage d'un régulateur (5) avec une valeur de consigne donnée et commandé par un capteur (4) en pression artérielle partielle en $O_2$, $PaO_2$, caractérisé en ce que:

a) les valeurs $FiO_2$ sont ajustées en même temps à une mémoire (14) et à un comparateur de minimisation (13) d'un dispositif de minimisation (12), la valeur du seuil superieur So du comparateur de minimisation (13) étant celle $FiO_2$ contenue jusque là dans la mémoire (14);
b) et un dispositif de commande (15) du dispositif de minimisation (12), qui comprend une bascule (16), un microprocessor (17) et une horloge (18), est raccordé au dispositif de réglage (19) du respirateur artificiel (1) pour y régler successivement les paramètres de respiration.

2. Respirateur artificiel selon la revendication 1, caractérisé en ce qu'à la bascule (16) sont raccordés par l'intermédiaire d'une porte OU (23), d'une part des capteurs (20) en parallèle pour la mesure d'autres données du patient par l'intermédiaire de comparateurs à fenêtre (21), et d'autre part le comparateur de minimisation (13).

3. Respirateur artificiel selon l'une des revendications 1 ou 2, caractérisé en ce chaque comparateur à fenêtre (21) est raccordé à un dispositif d'alarme (22).

4. Respirateur artificiel selon l'une des revendications 1 à 3, caractérisé en ce qu'un comparateur à fenêtre (25) en $FiO_2$ avec un dispositif d'alarme (26) est raccordé à l'entrée du comparateur de minimisation (13).

5. Respirateur artificiel selon l'une des revendications 1 à 4, caractérisé en ce qu'un dispositif d'alarme (24) est raccordé au dispositif de commande (15).

## Claims

1. A respiratory installation with a respirator controlled by patient values, wherein respirator gas with a definite $FiO_2$ (i.e. inspiration concentration of oxygen) is supplied to the respirator (1) from a mixer (7), which receives its adjusting values from a regulator (5) which is controlled by way of: an element (4), for detecting $PaO_2$ (i.e. the partial pressure of oxygen), on the patient (3); and an inputted pressure of oxygen), on the patient (3); and an inputted rated value characterised in that a) the $FiO_2$ values are simultaneously fed to a memory (14) and a minimisation comparator (13) of a minimisation apparatus (12), the upper threshold value So of the minimisation comparator (13) being the prevailing $FiO_2$ value held by the memory (14), and b) a control device (15) of the minimisation apparatus (12), which control device comprises a flip-flop (16), a microprocessor (17) and a timer (18), is connected to an adjusting device (19) of the respirator (1) in order to adjust consecutive respiration parameters at the respirator.

2. A respiratory installation according to claim 1, characterised in that detecting elements (20) are connected in parallel to the flip-flop (16) by way of an OR gate (23) for measuring additional patient data by way of window comparators (21) and the minimisation comparator (13).

3. A respiratory installation according to claims 1 and 2, characterised in that the window comparators (21) are each connected to a respective warning device (22).

4. A respiratory installation according to one of claims 1 to 3, characterised in that an $FiO_2$ window comparator (25) with a warning device (26) is connected to the input of the minimisation comparator (13).

5. A respiratory installation according one of claims 1 to 4, characterised in that an alarm (24) is connected to the control device (15).

Fig.1

0022144

Fig. 2

0 022 144